# EUROPEAN PATENT APPLICATION

(11) **EP 4 349 965 A1**
(43) Date of publication of application: **10.04.2024**
(21) Application number: 22816510.6
(22) Date of filing: 07.06.2022
(51) Int. Cl.: C12N 5/071, C12N 11/02, B01J 19/00

(54) **NANOVESICLE REACTOR AND MANUFACTURING METHOD THEREFOR**

(30) Priority: 04.06.2021 KR 20210072646; 03.06.2022 KR 20220068477
(71) Applicant: Institute for Basic Science, Yuseong-gu Daejeon 34126 (KR); Ulsan National Institute of Science and Technology, Ulju-gun, Ulsan 44919 (KR)
(72) Inventor: CHO, Yoon-Kyoung, Ulju-gun Ulsan 44920 (KR); SUMIT, Kumar, Ulju-gun Ulsan 44919 (KR)
(74) Representative: Kraus & Lederer PartGmbB
(86) International application number: PCT/KR2022/007994
(87) International publication number: WO 2022/255851

(57) **Abstract**

The present invention relates to a nano-vesicle reactor and a method for producing the same. Specifically, the present invention provides a composition for producing a nano-vesicle reactor, characterized by comprising a first vesicle containing a first receptor on its surface and a second vesicle containing a second receptor on its surface, wherein the first receptor and the second receptor are bound with each other through a ligand system so that the first vesicle and the second vesicle are fused to form the nano-vesicle reactor ; and a method for producing the above nano-vesicle reactor. The nano-vesicle reactor of the present invention can be utilized in production of energy inside a cell through cellular enzymatic reaction, a drug delivered system, and a diagnostic system.

## Description

### BACKGROUND OF THE INVENTION

### Field of the Invention

The present invention relates to a nano-vesicle reactor and a method for producing the same, and more specifically is directed to a nano-vesicle reactor that easily controls chemical reaction occurring inside a living cell using a modified nano-vesicle.

### Description of the Related Art

A life activity is maintained by movement of substances or energy within the body. A chemical reaction in vivo is controlled by the presence of a cell organelle or a core structure of the cell for accommodating enzymes or auxiliary factors responsible for various catalytic reactions in vivo. Recently, research has attempted to simulate biocatalytic reaction by artificially synthesizing the cell organelle.

The cell organelle can be said to be an organ of the cell, and specific example thereof includes mitochondria responsible for cellular respiration and lysosome responsible for a digestive system within the cell. A vesicle is a cell organelle surrounded by a fat bilayer and distinguished from the cytoplasm, and is responsible for storing, transporting, or digesting products, by-products, and excretions of the cell.

The cell organelle is divided into microscopic spaces that carry out complex biochemical reaction, and inside the spaces, there are various enzymes that perform important cellular functions. Among them, exosome is an extracellular vesicle with a diameter of 50 to 200 nm secreted by the cell and is known to serve as a carrier for signal transmission between the cells. The exosome has various information on parent cells such as a protein, a fat, RNA, and a growth factor, so that it can be used as a diagnostic marker for diseases, and a study for using as a drug delivery system due to its biocompatible characteristics is being actively conducted. However, even though the study on composition and function of the exosome has been actively conducted, there were still limitations in the technology for isolating and purifying the exosome because the exosome contains a large number of biomarkers, including a protein, a gene, and a nucleic acid.

Further, the factors such as intracellular mass transfer efficiency, stability, and biocompatibility must be considered in order to apply the artificial cell organelle such as the exosome to the living cell.

### SUMMARY OF THE INVENTION

Accordingly, the purpose of the present invention is to provide a nano-vesicle reactor capable of controlling function of a cell using modification and fusion of the nano-vesicle so as to solve the technical problem of requiring a more stable and efficient method of delivering an intracellular substance to a living cell.

According to an embodiment of the present invention, the purpose is to provide a nano-vesicle reactor capable of generating energy inside a cell using a nanoreactor platform technology.

According to an embodiment of the present invention, the purpose is to provide an effective drug delivery system using fusion of a nano-vesicle.

In order to solve the above-described technical problems, the present invention provides a composition for producing a nano-vesicle reactor, characterized by comprising a first vesicle containing a first receptor on its surface and a second vesicle containing a second receptor on its surface, wherein the first receptor and the second receptor are bound with each other through a ligand system so that the first vesicle and the second vesicle are fused to form the nano-vesicle reactor.

In an embodiment of the present invention, the first receptor and the second receptor may be independently with each other selected from the group consisting of CD9, CD63, CD81, CD82, CD147, CD107a, CD151, CD13, CD26, ITGA3, ITGA6, ITGB1, ITGB4, ADAM10, CD31B, EGFR, EpCAM, HSP70, HSP9, PSMA, and PSA.

In an embodiment of the present invention, the ligand system may contain a coordination-bonding compound.

In an embodiment of the present invention, the coordination-bonding compound may be an aromatic compound containing a polar substituent group.

In an embodiment of the present invention, the polar substituent group may include a hydroxyl group or a carboxylic acid group.

In an embodiment of the present invention, the ligand system may further form a supramolecular complex with multivalent metal ion.

In an embodiment of the present invention, the first vesicle and the second vesicle may be an extracellular vesicle.

In an embodiment of the present invention, the first vesicle and the second vesicle may contain different enzymes with each other.

In an embodiment of the present invention, the first vesicle and the second vesicle may have an average diameter of 10 to 1000 nm.

Also, the present invention provides a nanoreactor-containing emulsion comprising the composition for producing the nano-vesicle reactor described above, in a water phase inside the emulsion.

In an embodiment of the present invention, the emulsion may have an average diameter of 2 to 100 *µ*m.

Further, the present invention provides a cell containing the above-described nano-vesicle reactor therein.

In an embodiment of the present invention, the nano-vesicle reactor may be used for generating energy.

In an embodiment of the present invention, the first vesicle contained in the nano-vesicle reactor may include ATP synthase, and the second vesicle may include bo3 oxidase.

In an embodiment of the present invention, the nano-vesicle reactor may further comprise a drug.

In addition, the present invention provides a method for producing a nano-vesicle reactor, comprising the steps of:
(S 1) preparing a first vesicle solution containing a first receptor on a surface of the vesicle; (S2) preparing a second vesicle solution containing a second receptor on a surface of the vesicle; (S3) preparing a mixed solution by mixing the first vesicle solution and the second vesicle solution; and (S4) mixing the mixed solution with a multivalent metal ion to fuse the first vesicle and the second vesicle.

In an embodiment of the present invention, the first vesicle solution and the second vesicle solution of the steps (S 1) and (S2) may further comprise the step of treating them with a coordination-bonding compound independently with each other.

The current invention offers an artificial cell organelle with the capacity to produce cellular energy through the reprogramming of an extracellular vesicle that usually serves as an information carrier between living cells. This organelle, once introduced into the cell, exhibits stability and can penetrate deep into tissues, establishing it as an exceptionally valuable drug delivery system. According to an embodiment of the present invention, the ATP can be directly synthesized inside the cell and delivered deep into diseased cells that lacks energy supply, thereby supplying the energy may repair the damaged cells.

According to an embodiment of the present invention, since the nano-vesicle reactor can further comprise a drug, it can be used as an effective drug delivery system.

### BRIEF DESCRIPTION OF THE DRAWINGS

FIG. 1 is a schematic diagram showing the principle of inducing fusion of exosome by reacting exosome (CEx) modified to have a catechol group on its surface with a metal ion according to the present invention.
FIG. 2 shows the results of confirming size characteristics of exosome, CEx, and FEx using NTA (Nanoparticle Tracking Analysis).
FIG. 3(A) is a TEM image showing sizes of CEx (core diameter = 115 ± 26 nm) and FEx (core diameter = 247 ± 33 nm). FIG. 3(B) is a graph showing size distributions of CEx (CEx-1 = 121 ± 8 nm; CEx-2 = 123 ± 5 nm) and FEx (265 ± 14 nm) through dynamic light scattering.
FIG. 4 shows the results of cobalt-calcein analysis for monitoring fusion of CEx.
FIG. 5 shows the results of confirming a degree of fusion by varying the types of polyvalent metal ions.
FIG. 6 shows a schematic diagram of the continuous step reaction of glucose oxidase (GOx) and HRP (horseradish peroxidase) enzyme inside FEx. The reaction was initiated by adding glucose, and H₂O₂ was produced through aerobic oxidation, which shows that resorufin was produced by oxidizing Amplex Red.

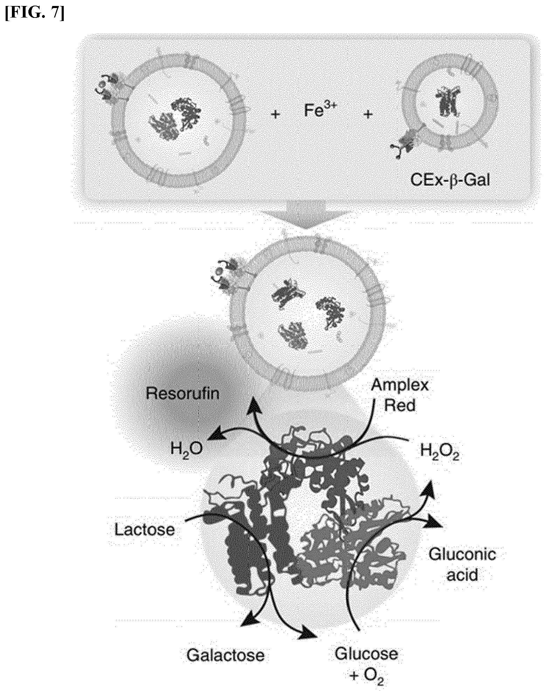

FIG. 7 is a schematic diagram showing catalytic action of three (3) enzymes encapsulated in a FEx nanoreactor. In the presence of lactose, β-galactosidase-catalyzed hydrolysis produces glucose and galactose. This aerobic GOx-catalyzed oxidation of the glucose produces gluconic acid and H₂O₂, which then catalyzes oxidation of Amplex red to produce resorufin.
FIG. 8 shows change in a fluorescence intensity of resorufin over time by two-enzyme cascades encapsulated into a FEx-GOx-HRP nanoreactor, which can confirm that the fluorescence intensity of resorufin gradually increases and reaches saturation after 80 minutes.
FIG. 9 shows change in a reaction velocity depending on lactose concentration by three-enzyme cascades encapsulated in the FEx-β-galactosidase-GOx-HRP nanoreactor.
FIG. 10 shows change in a relative activity of enzymes encapsulated inside a FEx nanoreactor (FEx-GOx-HRP) and free enzymes in a solution, and is graphs showing the results of enzyme characteristics for a storage stability (FIG. 10(A)), a thermal stability (FIG. 10(B)), and a cycle characteristic (FIG. 10(C)).
FIG. 11 relates to Experimental Example 3, which evaluated catalytic function within the platforms compartmentalized according to the present invention. FIG. 11(A) is a schematic diagram showing the catalytic conversion of proRho to free rhodamine inside a cell using a FEx-Pd-proRho nanoreactor produced by fusing a palladium nanoparticle (Pd), and FIG. 11(B) shows a representative TEM image of FEx-Pd-proRho (Pd nanostructure with a size of ~6 nm, Scale bar 10 nm).
FIG. 12(A) shows fluorescence emission spectrum (λEx = 498 nm) for demasking rhodamine in FEx and CEx, and FIG. 12(B) shows change (at 527 nm) in fluorescence intensity (%) of demasking reaction as a function of time in FEx-Pd-proRho and CEx-proRho.
FIG. 13(A) is a schematic diagram showing catalytic conversion of a pro-drug into a drug using a FEx-Pd-proFU nanoreactor, and shows activation of the anticancer drug 5-fluorouracil (pro-FU) using the FEx-Pd-proFU nanoreactor. FIG. 13(b) is a graph showing the results of cell viability of a MCF-10A cell after culturing the FEx-Pd-proFU and CEx-proFU nanoreactors at various concentrations (10 µg/mL, 30 µg/mL, 60 µg/mL, and 90 µg/mL).
FIG. 14 is a schematic diagram showing an artificial cell organelle (FEx-1) for generating energy according to Example 2 of the present invention. The energy can be supplied even to a cell in hypoxic condition by producing ATP with the artificial cell organelle that is created by fusing exosome (ATPsyn-CEx-GOx) loaded with ATP synthase and glucose oxidase and exosome (bo3Oxi-CEx-HRP) loaded with bo3 oxidase and HRP enzyme, on/inside an exosome membrane, respectively.
FIG. 15 is graphs showing the results of observing a pH gradient over time (FIG. 15 (A)) and ATP production over time (FIG. 15 (B)) to check whether energy is generated inside a FEx-1 nanoreactor after absorption into a cell.
FIG. 16(A) shows ATP activity after FEx-1 was absorbed into a hypoxia-induced cell. FIG. 16(B) shows quantification of intracellular ROS levels in a FEx-1 nanoreactor treated with different concentrations.

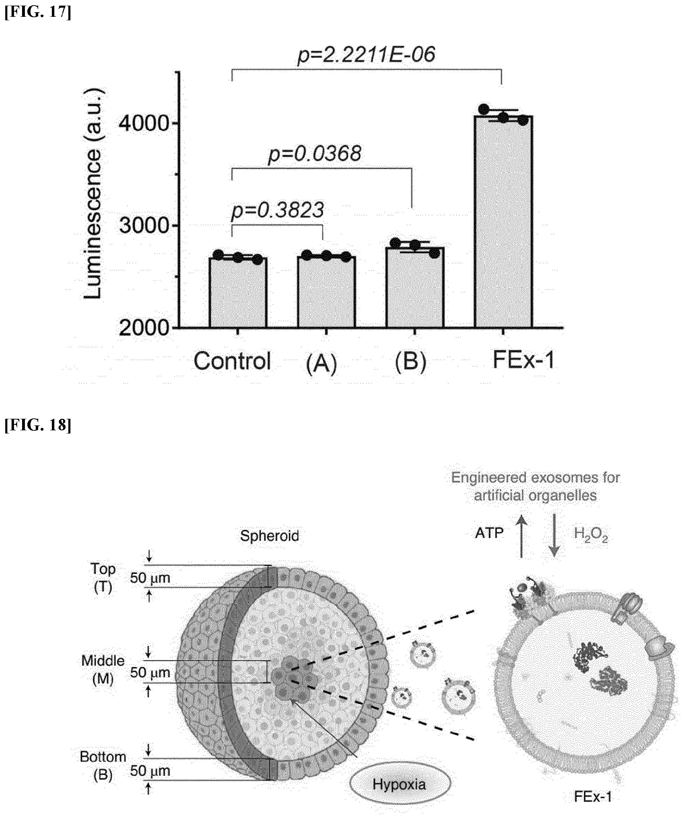

FIG. 17 is a graph showing a degree of luminescence of ATP synthesized by fused exosomes or individual exosome according to Example 2 of the present invention and Comparative Example 2.
FIG. 18 shows a structure of the three-dimensional multicellular spheroid which is very similar to an in vivo tissue containing a hypoxic core, and is a schematic diagram showing that FEx can penetrate into the spheroid and control cellular redox under the hypoxic condition through ATP production.
FIG. 19 is a graph showing the results of measuring ATP activity level of the spheroid after being treated with fused exosomes (FEx-1) and cultured for 24 hours.
FIG. 20 is a graph showing the results of cell viability analysis of the spheroid using calcein-AM (acetoxymethyl).
FIG. 21 is a graph showing the results of measuring active oxygen (ROS) level of the spheroid after treatment with fused exosomes (FEx-1).

### DETAILED DESCRIPTION OF THE INVENTION

Hereinafter, according to the present invention, a composition for producing a nano-vesicle reactor, an emulsion containing the nano-vesicle reactor, a cell containing the nano-vesicle reactor, and a method for producing the nano-vesicle reactor will be described in detail.

Herein, unless otherwise defined, all technical and scientific terms in the specification of the present invention have meanings commonly understood by a person who has an ordinary knowledge in the technical field to which the present invention pertains.

The terminology used in the detailed description of the specification is merely to effectively describe particular embodiments and is not intended to limit the invention thereto.

Further, the terms such as first, second, A, B, (a), and (b) may be used when describing the constitutive elements of the present invention. These terms are only used to distinguish the constitutive elements from other constitutive elements, and the nature, sequence, or order of the constitutive elements is not limited by the terms.

Further, unless otherwise specified in the specification, the unit of additives may be weight percent.

In addition, as used herein, a singular form may also be intended to include a plural form, unless the context clearly dictates otherwise.

Furthermore, in case a certain part "includes" a certain constitutive element, this means that it may further comprise other constitutive elements rather than excluding the other constitutive elements, unless specifically stated to the contrary.

In the specification, descriptions of known effects and configurations that may unnecessarily obscure the gist of the present invention are omitted.

In the specification of the present invention, the "nano-vesicle reactor" may refer to a platform that performs the function of inserting a nanoparticle into a cell or a living body and inducing chemical reaction that may or may not occur in the living body. In the specification, the "nanoreactor" or "vesicle nanoreactor" can be interpreted as having the same meaning.

In the specification of the present invention, the 'receptor' is a molecule bound to a surface of a vesicle membrane and may refer to a molecule that selectively binds to a specific substance.

In the specification of the present invention, the "ligand system" may refer to a certain substance that specifically binds to a receptor.

Artificial cell organelles, which are artificially synthesized and utilized, are still in the early stage of development. Among them, exosome, which is an extracellular vesicle, has a nano-size and is a bio-derived substance, and thus a research is continuously conducted due to the advantage in that the exosome is widely used for delivering a drug, a gene, and a vaccine. However, in order to apply the exosome to a living cell, it must be able to maintain activity inside the cell for a long period of time and not affect viability of the cell.

The inventor of the present invention has completed the present invention by confirming that the extracellular vesicle, which functions as the information carrier between the living cells, was reprogrammed to combine two types of the nano-vesicle reactors through chemical reaction on a droplet-based microfluidic reactor, thereby causing occurrence of the chemical reaction and fusion between the internal substances.

The present invention can provide a fused nano-vesicle reactor capable of easily controlling biocatalytic reaction through fusion reaction of the nano-vesicle reactors having different enzymes or reactants inside the vesicle and a membrane protein, and generating energy inside a living cell by containing enzymes that enable to synthesize ATP, an energy source of the cell.

Hereinafter, a composition for producing a nano-vesicle reactor according to the present invention will be described in detail.

Specifically, the composition for producing the nano-vesicle reactor can be characterized by comprising a first vesicle containing a first receptor on its surface and a second vesicle containing a second receptor on its surface, wherein the first receptor and the second receptor are bound with each other through a ligand system so that the first vesicle and the second vesicle are fused to form the nano-vesicle reactor. In this case, in an embodiment of the present invention, the first receptor and the second receptor may be the same as or different from each other. The same or different receptors can be selected if necessary depending on the purpose of the present invention.

Specifically, the first receptor and the second receptor may be a cell surface protein. Concrete examples of the cell surface protein may be selected from the group consisting of CD9, CD63, CD81, CD82, CD147, CD107a, CD151, CD13, CD26, ITGA3, ITGA6, ITGB1, ITGB4, ADAM10, CD31B, EGFR, EpCAM, PSMA, and PSA, but is not particularly limited thereto.

Further, in an embodiment of the present invention, the first receptor and the second receptor may be a cancer-specific antibody.

The ligand system may contain a receptor binding unit that interacts with the receptor through a specific non-covalent bond. Examples of the non-covalent bond may be a hydrogen bond, an ionic bond, a hydrophobic interaction, or a van der Waals force. The receptor binding unit may be anti-CD9, anti-CD63, anti-CD81, anti-CD82, anti-CD147, anti-CD107a, anti-CD151, anti-CD13, anti-CD26, anti-ITGA3, anti-ITGA6, anti-ITGB1, anti-ITGB4, anti-ADAM10, anti-CD31B, anti-EGFR, anti-EpCAM, anti-PSMA, and anti-PSA, which can form the specific non-covalent bond with the receptor, but are not necessarily limited thereto. In this case, the "anti-" may refer to a molecule or molecule group containing one or more epitopes that form a specific bond with the cell surface protein.

Further, the composition for producing the nano-vesicle reactor according to the present invention may comprise a coordination-bonding compound in the ligand system. In this case, in an embodiment of the present invention, the coordination-bonding compound may be an aromatic compound containing a polar substituent group. Specifically, it may be a phenol-based compound. More specifically, it may be a phenol-based compound containing one or more hydroxyl or carboxylic acid groups, and may be, for example, phenol, catechol, protocatechuic acid, tannic acid, gallic acid, lignin, ellagic acid, etc., but is not limited thereto.

Furthermore, the ligand system may further contain a multivalent metal ion. Specifically, the multivalent metal ion may be an aluminum ion, a titanium ion, a cesium ion, a zinc ion, or an iron ion. More specifically, it may be Al³+, Ti⁴+, Ce³+, Zn²+, or Fe³+, and more specifically, may be Al³+, Zn²+, or Fe³+.

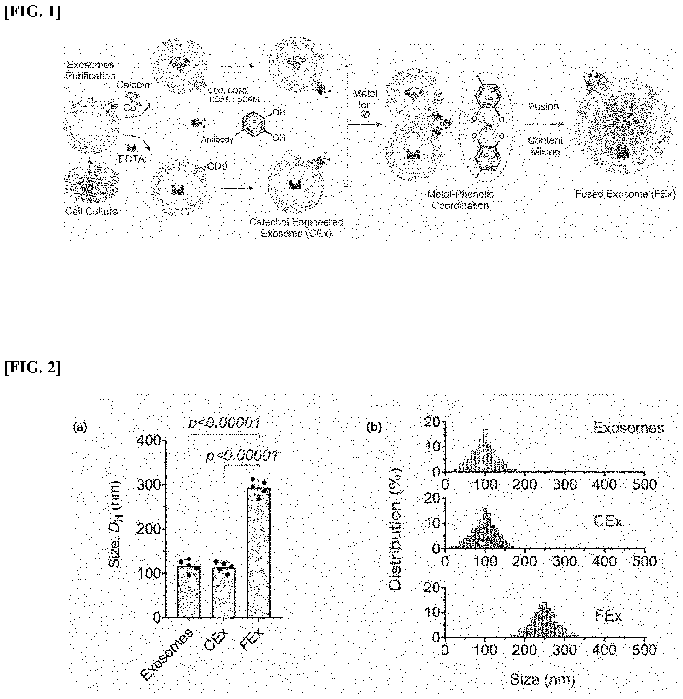

According to this, a complex of the receptor binding unit and the coordination-bonding compound may form a coordination bond with the multivalent metal ion. A polar substituent group of the coordination-bonding compound may include a hydroxyl group or a carboxylic acid group, and may easily form a metal-phenolic coordination bond together with the multivalent metal ion. As shown in FIG. 1, the fusion of the first vesicle and the second vesicle can be induced through the metal-phenolic coordination bond.

In the present invention, the vesicle consists of a structure of a phospholipid double membrane identical to that of the cell membrane, and is not particularly limited as long as its outer membrane can stably maintain the substances inside the vesicle. An average diameter of the vesicle may be 10 to 1000 nm, or 30 to 500 nm. The vesicle may be a naturally derived or artificially synthesized nano-vesicle.

In a non-limiting embodiment of the present invention, the nano-vesicles may be various extracellular vesicles derived from a living organism.

In an embodiment of the present invention, the fusion of the two types of the nano-vesicles may be performed inside a droplet-based microfluidic reactor having an average diameter of 10 *µ*m or less. In this case, the two types of the nano-vesicles may be exosome, extracellular vesicle other than the exosome, or a combination thereof. Specifically, they may, for example, be i) exosomes that are different from each other; ii) extracellular vesicles other than the different exosomes; or iii) exosome and extracellular vesicle other than the exosome.

The droplet-based microfluidic reactor is used to synthesize a new functional material or analyze a biological material with high sensitivity by forming multiple droplets on a microfluidic chip using two immiscible fluids such as water and oil and utilizing these multiple small droplets having such separated sections with a biochemical reactor. According to an embodiment of the present invention, chemical reaction of the substances inside each nano-vesicle can be derived through fusion of the nano-vesicles using the droplet-based microfluidic reactor.

In an embodiment of the present invention, the number of the vesicle per the droplet-based microfluidic reactor may be 200 or less, specifically 100 or less, and more specifically 60 or less. According to this, the biocatalytic reaction can be successfully controlled by the fusion reaction of the nano-vesicles with different enzymes inside the nano-vesicle and on the membrane protein.

In a non-limiting embodiment of the present invention, the extracellular vesicle may be exosome that can stably perform enzyme cascade reaction and have excellent targeting ability. The main advantage of the exosome nanoreactor is that the membrane protein acts as a permeable shell to facilitate flow of a chemical reagent, and that leaching of the loaded enzymes is prevented for better biocatalytic performance to facilitate deep penetration into a tissue.

In an embodiment of the present invention, the first vesicle and the second vesicle may contain the same receptor and contain different enzymes. In this case, there may be two or more types of enzymes or three or more types of enzymes, and the nano-vesicle reactor may perform the cascade reaction using a plurality of enzymes.

Concrete examples of the enzymes may include NADH dehydrogenase, acetylcholinesterase, alcohol dehydrogenase, bacteriorhodopsin, photosystem II, etc., without being limited thereto, and may be applied in various manners if necessary as long as they can be encapsulated inside the vesicle.

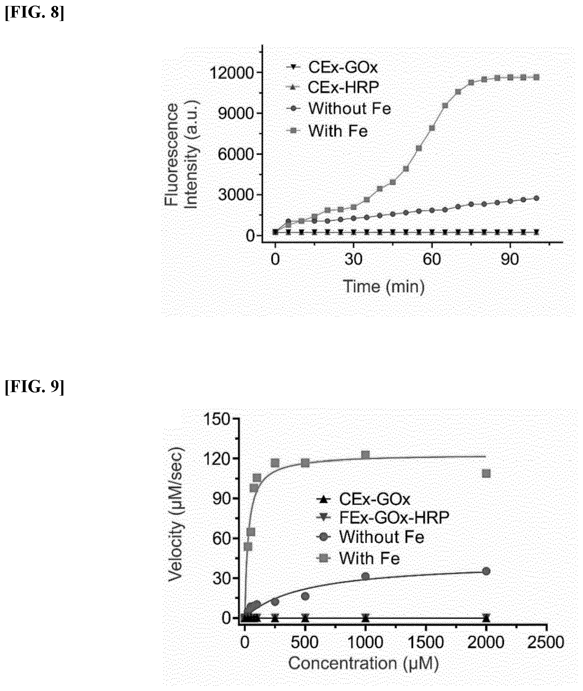

FIG. 9 shows the results of three catalyst-cascade reaction, and specifically shows three enzyme cascade encapsulated in a FEx-β-galactosidase-GOx-HRP nanoreactor. In the presence of lactose as a substrate, β-galactosidase (23.6 µg/mg) catalyzed hydrolysis of the lactose to produce glucose and galactose. Oxidation of the produced glucose with an aerobic GOx catalyst (31.2 µg/mg) produced gluconic acid and H₂O₂, and HRP (18.9 µg/mg) was used to oxidize Amplex Red to form resorufin. Lactose-dependent change in fluorescence of the resorufin was monitored using a fluorescence microscopy. The reaction velocity (µM/sec) was plotted against lactose concentration (µM) to calculate a kinetic constant (Km and Vmax). The Km values for the FEx-β-galactosidase-GOx-HRP nanoreactor and FEx-GOx-HRP having CEx-β-galactosidase were 28.78 × 10⁻⁶ M and 489.65 × 10⁻⁶ M, respectively, and the Vₘₐₓ values for the FEx-β-galactosidase-GOx-HRP nanoreactor and FEx-GOx-HRP having CEx-β-galactosidase were 123.16 × 10⁻⁶ M/min and 43.06 × 10⁻⁶ M/min, respectively.

Further, the present invention can provide an emulsion comprising the composition for producing the nano-vesicle reactor described above, in a water phase of the emulsion. In this case, the emulsion may have an average diameter of 2 to 100 *µ*m or 1 to 50 *µ*m.

Furthermore, the present invention can provide a cell containing the composition for producing the nano-vesicle reactor described above. As a non-limiting example, the nano-vesicle reactor may include ATP synthase in the first vesicle and bo3 oxidase in the second vesicle. Through this, the nano-vesicle reactor can be used to produce energy and can provide an advantage of being used as a nano pill that supplies energy to a cell damaged by hypoxia.

In an embodiment of the present invention, the nano-vesicle reactor may be absorbed into the cell for its operation. Specifically, the nano-vesicle reactor may be absorbed into the cell through endocytosis, but without being limited to this, may be absorbed into the cell through various mechanisms.

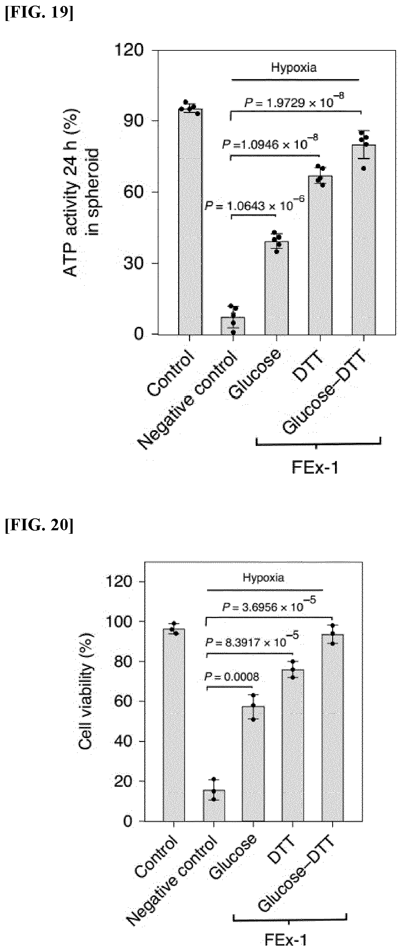

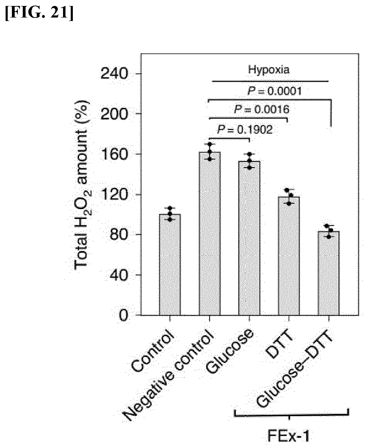

The nano-vesicle reactor according to the present invention can penetrate deep into a core portion of a multicellular spheroid containing a hypoxic core. As shown in FIGS. 19 to 21, it was confirmed that artificial exosome can penetrate deep into the inside of a breast cancer-simulating spheroid. This suggests possibility of the artificial exosome to be used as a nano pill that supplies energy to the cell damaged by hypoxia, thereby enabling to provide a solution to ATP deficiency in the center of a tumor.

The cell containing the nano-vesicle reactor may be bacteria, a plant, or an animal-derived cell including a human.

In addition, the present invention can provide a method for producing a nano-vesicle reactor, comprising the steps of: (S1) preparing a first vesicle solution containing a first receptor on a surface of the vesicle; (S2) preparing a second vesicle solution containing a second receptor on a surface of the vesicle; (S3) preparing a mixed solution by mixing the first vesicle solution and the second vesicle solution; and (S4) mixing the mixed solution with a multivalent metal ion to fuse the first vesicle and the second vesicle.

In an embodiment of the present invention, the first vesicle solution and the second vesicle solution of the steps (S1) and (S2) may further comprise the step of treating them with a coordination-bonding compound independently with each other.

The present invention will be described in more detail through Examples below. However, the following Examples are nothing but a reference for explaining the present invention in detail, and the present invention may be implemented in various forms without being limited thereto.

### Preparation Example 1. Preparation of exosome (CEx) modified with catechol

Human mammary epithelial cell MCF-10A (cat#CRL-10317, American Type Culture Collection (ATCC)) was seeded into a T75 cell culture flask (Corning, NY, USA) containing 18 ml of Dulberico's modified medium (DMEM/F-12; cat# 11330-032) that was supplemented with 5% of a horse serum (Invitrogen #16050-122), 20 ng/ml of an epidermal growth factor (EGF, cat# AF-100-15, Peprotech), 0.5 mg/ml of hydrocortisone (cat# H0888, Millipore), 10 µg/ml of insulin (cat# I1882, Millipore), 1% of an antibiotic/antifungal agent (100 U/ml penicillin and 100 mg/ml streptomycin), at a density of 5×10⁶ cells per the flask. The cell was cultured until 60-70% confluence at 37 °C in an incubator having a humidified atmosphere and containing 5% CO₂, followed by being washed twice using 1x phosphate-buffered saline (PBS), and was washed again using a RPMI (Roswell Park Memorial Institute) medium (Gibco, Thermo Fisher Scientific) supplemented with 10% of Exo-Free FBS (Systems Biosciences Inc., CA, USA) and 1% of an antibiotic/antifungal solution. Thereafter, the cell was incubated in an incubator of 37 °C for 48 hours, and then a supernatant of the cell culture was collected, and exosome was immediately isolated by ultracentrifugation.

A dopamine hydrochloride (1 mM, cat# 62-31-7, Sigma Aldrich) solution and 2 mM of a disuccinimidyl carbonate solution were prepared in 1 mL and 15 mL of dimethyl formamide, respectively. The dopamine hydrochloride solution was added dropwise to 2mM of the disuccinimidyl carbonate solution for 30 minutes with stirring, and then 2mM of Et₃N was slowly added thereto. After 1 hour, a solvent was partially evaporated and 1N HCl was added. Finally, a product was extracted from this mixture using EtOAc. Thereafter, in order to bind an anti-CD9 antibody, catechol-NHS (1 mg) was dissolved in a minimal amount of dimethyl formamide and added to a solution of a CD9 antibody (BioLegend, Mouse Monoclonal) in a PBS (0.2 mg/mL). A molar ratio of the reagent to the antibody solution was adjusted to >10. After 2 hours, the product was purified by dialysis against the PBS. MCF-10A-derived exosome was incubated with catechol-anti-CD9 (500 ng/mL) at 37 °C for 1 hour to obtain catechol-engineered exosome (CEx). The final product could be obtained by purification from the reaction mixture with ultracentrifugation (120,000 × g and 4 °C for 2 hours).

### Example 1. Preparation of fused exosome (FEx)

A mixture of 50 µM of calcein, 1 µM of CoCl₂, 10 mM of MOPS (pH 7.4), and 10 µg/mL of the CEx prepared according to Preparation Example 1 above was sonicated at a room temperature for 3 minutes to encapsulate a complex of the calcein and the Co²⁺ inside the CEx (CEx-1). Then, 10 µM of EDTA, 10 mM of MOPS (pH 7.4), and 10 µg/mL of the CEx prepared according to Preparation Example 1 were mixed to encapsulate EDTA inside the CEx (CEx-2). Both of the CEx-1 and the CEx-2 were centrifuged twice at 120,000 × g for 1 hour to remove an excess amount of the EDTA and the cobalt calcein. A bright orange pellet was resuspended in 0.6 mL of a buffer containing 10 mM of MOPS (pH 7.4), and an aqueous phase of the CEx suspension (3 × 10⁹ particles/mL) and FeCl₃·6H₂O (40 ng/mL) solution was injected to a microfluidic device to perform fusion of the CEx-1 exosome and the CEx-2 exosome.

### Example 2. Preparation of fused exosome FEx-1 (ATPsyn-bo3Oxi-FEx-Gox-HRP)

To a mixture of 60 µL of 10% sodium cholate, 300 µL of a MCF-10A-derived exosome suspension (5 × 10⁹ ± 2.6 × 10⁸ particles/mL calculated using NTA, 87 µg calculated using BCA analysis) and 200 µL of MOPS buffer (pH 7.4), 100 µL of ATP synthase and bo3 oxidase (1 mg/mL calculated using the BCA analysis) were added separately for each of the two reactions. The mixture was stirred at 4 °C for 15 minutes and passed through a gravity column containing a Sephadex G-50 resin equilibrated with the MOPS buffer at a room temperature. The cloudy fraction was transferred to a new ultracentrifuge tube, and the exosome suspension was washed twice, and centrifuged in a Ti45 fixed-angle rotor (Beckman Coulter) at 120,000 × g and 4 °C for 2 hours to prepare fused exosome FEx-1 (ATPsyn-bo3Oxi-FEx-Gox-HRP) by removing excess ATP synthase.

The exosome pellet was resuspended in 200 µL of pre-filtered PBS and stored at 4 °C for using it immediately or at -80 °C for storing it for a long period of time. An amount of the ATP synthase and the bo3 oxidase fused to the exosome was measured to be 23 ng/µg and 17 ng/µg, respectively.

### Example 3. Preparation of fused exosome FEx-Pd-proRho

Exosome (CEx-Pd) encapsulated in CEx was prepared by adding palladium nanoparticles (Pd NPs) to a mixture of 10 µg/mL of the CEx prepared according to Preparation Example 1, and exosome (CEx-proRho) loaded with bis-allyloxycarbonyl-protected rhodamine (proRho) was prepared in the same manner. Fusion between the CEx-Pd exosome and the CEx-proRho exosome was performed to prepare fused exosome (FEx-Pd-proRho).

### Comparative Example 2. Preparation of exosome directly loaded with enzyme

### (A) Preparation of exosome encapsulated therein using all of GOx, Hrp, ATP synthase, and bo3 oxidase

An exosome suspension (250 µL) with a final protein concentration of 3.6 µg/mL was cooled on an ice and 0.2% of a saponin solution was added thereto. The mixture was sonicated in a capped glass vial at a frequency of 42 kHz and an electric power of 100 W for 2 minutes using a FS30D bath-typed ultrasonic processor (Fisher Scientific). After shaking at a room temperature for 20 minutes, the enzyme-loaded exosome was ultracentrifuged (at 120,000 × g and 4 °C for 2 hours) to remove a free enzyme, and then was quantified using a Pierce^{™} BCA protein assay kit (Thermo Fisher Scientific).

### (B) Preparation of exosome with GOx, Hrp, ATP synthase, and bo3 oxidase sequentially encapsulated therein.

The same preparation procedure as the above (A) was performed, except that all of the enzymes (GOx, HRP, ATP synthase, and bo3 oxidase) were encapsulated sequentially with each wash between the steps.

### [Experimental Example 1] Confirmation of fused exosome characteristics

### 1-1. Size distribution of exosome measured using Dynamic Light Scattering (DLS)

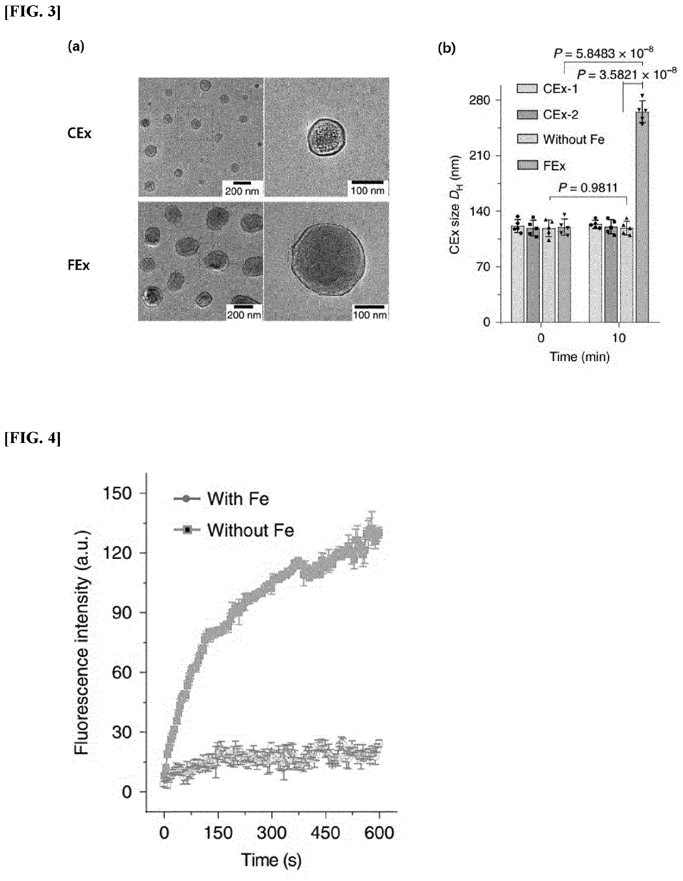

Size distribution of exosome was recorded by analyzing movement velocity distribution of the exosome due to dynamic fluctuation in a scattered light intensity at a fixed angle using a Malvern Zetasizer (Nano ZS). A hydrodynamic radius of the exosome due to Brownian motion was calculated using the Stock-Einstein equation. The exosome solution was diluted in particle-free PBS (1000×, passed through a 200 nm filter). About 1 mL of the sample was vortexed for 30 seconds and quickly loaded into a disposable cuvette so as to avoid its agglomeration. Size distribution of the exosomes of CEx and FEx was measured by at least three independent experiments. The results thereof were shown in FIG. 3.

### 1-2. Analysis of calcein-Co²⁺

In order to confirm that the fused exosome (FEx) is not a simple conjugation but a mixture of internal contents, the CEx-1 and the CEx-2 were fused separately to perform analysis of calcein-Co²⁺. The results thereof were shown in FIG. 4. Plotting the fluorescence intensity to time showed that a signal was increased approximately 10-folds after fusion and saturated within 10 minutes. This is because, as mixing between the membrane and the internal substance occurs, EDTA encapsulated inside the CEx-2 preferentially binds to Co²⁺ encapsulated inside the CEx-1, thereby preventing calcein from causing fluorescence. It could be confirmed from the above that mixing of the contents occurred.

### [Experimental Example 2] Evaluation of multi-enzyme cascade operation in fused exosome nanoreactor

### Two-enzyme cascade reactions:

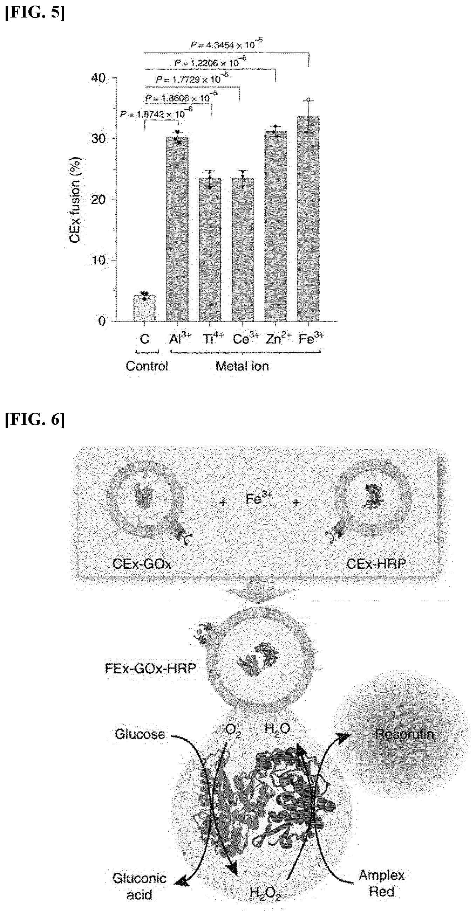

As shown in the schematic diagram of FIG. 6, after exosomes, which were loaded with glucose oxidase (GOx) and HRP enzyme, respectively, were fused, a multiple enzyme biocatalyst-sequential step reaction was carried out inside a fused exosome nanoreactor (FEx-GOx-HRP). By adding glucose, hydrogen peroxide and gluconic acid were produced through aerobic oxidation, and the hydrogen peroxide produced around the HRP acted as a substrate, allowing the HRP to catalyze oxidation of Amplex Red to produce resorufin, a fluorescent product.

Enzyme activity and kinetics of the FEx-Gox-HRP nanoreactor were monitored. Enzyme encapsulation efficiency was determined by a fluorescence spectrophotometry using calibration curves for enzymes labeled with different dyes. A reaction velocity of the enzyme biocatalytic cascade was measured using a fluorescence intensity of the resorufin at 563 nm using a microplate reader (TECAN Infinite M200PRO). In order to determine Vmax and KM of the FEx nanoreactor, the standard Michaelis-Menten enzyme kinetics equation was used, and GraphPad Prism 8.0.1 was used. It was shown that a Michaelis-Menten constant (Km) value of the FEx-GOx-HRP nanoreactor decreased up to 16-folds and a maximum reaction velocity (Vₘₐₓ) of the FEx-GOx-HRP nanoreactor increased up to 3-folds, compared to the homogeneous mixture of CEx-GOx and CEx-HRP.

### 1) Evaluation of storage stability

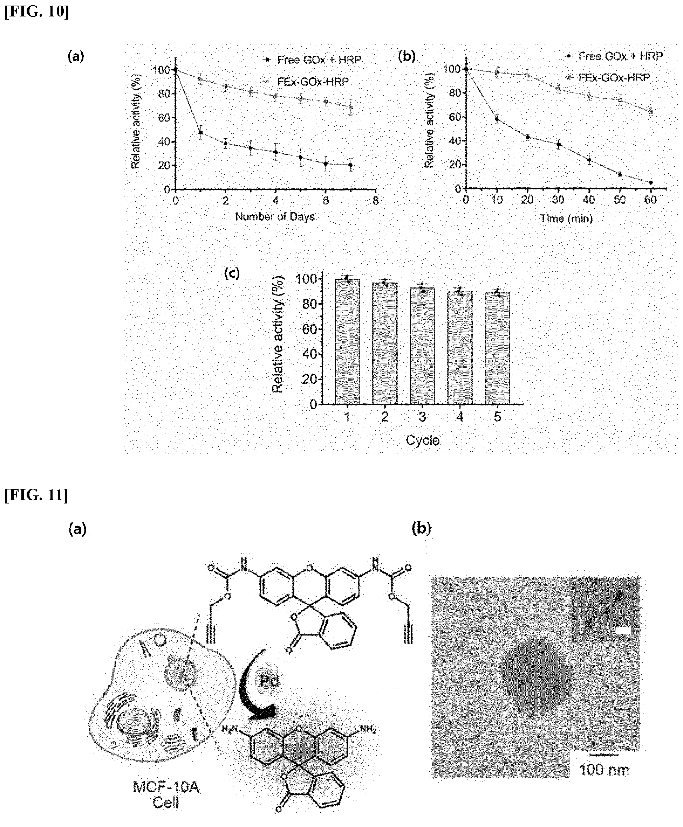

In order to determine change in the relative activity of free GOx and HRP in the solution and enzyme encapsulated inside the FEx nanoreactor (FEx-GOx-HRP), after storage at a room temperature (25 °C) for several days, the free enzyme (GOx + HRP) and FEx-GOx-HRP were stored in PBS at a room temperature (25 °C) to measure a storage stability. The activity was measured for 7 days to determine the stability during storage. The results thereof were shown in FIG. 10(A). It could be confirmed that in the case of the free enzyme, the relative activity was decreased to 20%, whereas the enzyme encapsulated inside the FEx nanoreactor maintained 69% of its activity even after 7 days.

### 2) Evaluation of thermal stability

After culturing at 60 °C for 1 hour, change in the relative activity of free GOx and HRP encapsulated in the solution and inside the FEx nanoreactor (FEx-GOx-HRP) were analyzed and the results thereof were shown in FIG. 10(B). The catalytic activity was measured every 10 minutes to determine a thermal stability. It was confirmed that in the case of the free enzyme, the relative activity was decreased to -5%, whereas the encapsulated enzyme showed 64% of the relative activity even after 1 hour of the culture.

### 3) Evaluation of recyclability

Change in the relative activity of the FEx-GOx-HRP system was analyzed after five repeated cycles, and the results thereof were shown in FIG. 10(C). It was confirmed that the initial activity of the FEx-GOx-HRP was maintained at more than 89% even after five repeated experiments.

### [Experimental Example 3] Bioorthogonal catalysis in fused exosome

### 3-1. Evaluation of catalytic function inside compartmentalization of fused exosome

In order to evaluate catalytic function inside the compartmentalization, as shown in FIG. 11, palladium-catalyzed deallylation was performed by a model bioorthogonal reaction to generate fluorescence from a fluorophore trapped in allylcarbamate inside a cell. Pd NP inside the FEx-Pd-proRho prepared by Example 3 was quantified with ICP-MS. The presence of Pd NP can be confirmed by the TEM of high-resolution shown in FIG. 11(B).

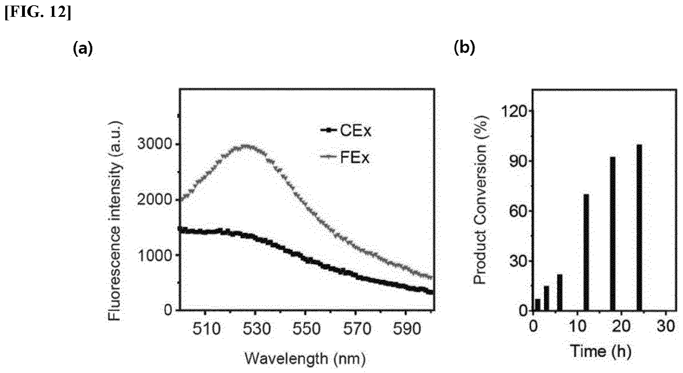

The catalytic characteristics of the nanoreactor were assessed by formation of the fluorescent product rhodamine monitored with a fluorometer. As shown in FIG. 12, the fluorescence intensity of the reaction solution was increased over time at 527 nm for the FEx-Pd-proRho, but showed little change for the CEx-proRho.

### 3-2. Intracellular catalysis of fused exosome

### 1) Confirmation of fluorescence image

Intracellular catalysis experiment was performed by culturing the FEx-Pd-proRho nanoreactor (30 µg/mL) together with a MCF-10A cell at 37 °C for 24 hours. A CLSM image of the MCF-10A cell showed green fluorescence due to catalytic conversion of proRho to fluorescent rhodamine, but no fluorescence signal was detected in the control experiment using CEx.

### 2) Flow cytometric analysis for precursor dye activation

MCF-10A human mammary epithelial cells (1 × 10⁵ cells) were seeded in a 6-well plate, and were cultured in DMEM supplemented with 5% of a horse serum (Invitrogen #16050-122), 20 ng/mL of an epidermal growth factor (EGF, cat# AF-100-15, Peprotech), 0.5 mg/mL of hydrocortisone (cat# H0888, Millipore), 10 µg/mL of insulin (cat# I1882, Millipore) and 1% of an antibiotic/antifungal agent (100 U/mL of penicillin and 100 mg/mL of streptomycin) at 37 °C and 5% CO₂. After 24 hours, a supernatant was removed and replaced with a fresh medium containing a suspension of FEX-Pd-proRho (30 µg/mL). In order to remove excess FEx-Pd-proRho, the cells were cultured for 24 hours and then washed with PBS. After washing the cells with the PBS, they were separated by culturing with a cell stripper solution (Corning^{®} 100 mL Cellstripper^{™}) for 20 minutes. Thereafter, the cells were harvested at 1200 rpm for 3 minutes and resuspended in the PBS using 2% of a fetal bovine serum (FBS). The cells were prepared for flow cytometry (CytoFLEX, Beckman Coulter Life) using FSC xV, SSC xV, FITC xV, APC xV setting. 50,000 events per a sample were recorded. The used excitation and emission wavelengths were 530/30 nm FITC for rhodamine, respectively. The data were analyzed using FlowJo software (FlowJo, LLC). Although not shown, the flow cytometry analysis confirmed that the fluorescence intensity signal was 100-folds higher after the reaction.

### 3) Evaluation of drug delivery system characteristics

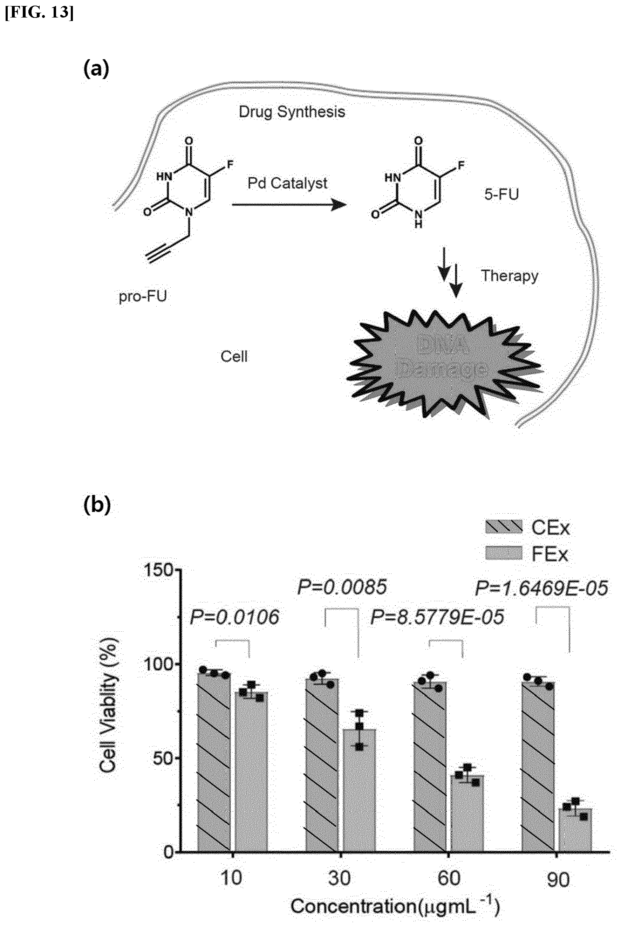

As shown in FIG. 13(A), conversion characteristics of a pro-drug into a drug were confirmed using the FEx-Pd-proFU nanoreactor. For bioorthogonal demasking of 5-fluorouracil (5-FU), fusion between CEx-Pd and masked inert therapeutic molecule 5-fluoro-1-propargyl uracil (proFU)]-loaded CEx was performed. This drug acts to disrupt cellular function by converting cytotoxic nucleotide metabolites through functionalization of the N1 position followed by inhibiting thymidylate synthase, and incorporating into RNA and DNA strands. In order to check the intracellular function/demasking of this drug, cytotoxicity was evaluated through live/dead cell analysis on a MCF-10A cell after culturing together with the FEx-Pd-proFU for 24 hours. The results thereof were shown in FIG. 13(B). It could be confirmed that the cell viability was dropped to 23% when cultured together with the FEx-Pd-proFU, whereas in the case of the CEx cells, the cell viability was 91%.

### [Experimental Example 4] Confirmation of energy generation of fused exosome

In order to confirm whether ATP was produced in exosome by direct loading of the fused exosome FEx-1 (ATPase-bo3Oxi-FEx-Gox-HRP) nanoreactor prepared according to Example 2 and the enzyme prepared according to Comparative Example 2, change in pH and ATP activity was measured.

### 4-1. Change in pH

The FEx-1 nanoreactor containing different enzymes (HRP, GOx, ATP synthase, and bo3 oxidase) was treated with glucose and DTT to initiate the enzymatic reaction. HPTS was encapsulated in FEx-1, and the protein was diluted to a final concentration of 137 µg/mL in a black 96-well plate and cultured at 30 °C for 5 minutes. Afterwards, 40 µM of coenzyme Q1 and 2 mM of DTT were added thereto so that the DTT reduces the Q1 to enable it to use in bo3 oxidase. One minute after addition of the DTT, glucose (300 nM) was added to the reaction mixture. Fluorescence spectrum was measured from 0 to 90 minutes using a spectrophotometer (TECAN, Morrisville, NC, USA) with excitation from 380 ± 5 to 480 ± 5 nm and emission from 512 ± 5 nm, and a pH was obtained from the calibration curve. The results thereof were shown in FIG. 9(A).

Proton generation inside the nanoreactor was confirmed by measuring a fluorescence signal of the HPTS (hydroxypyrene-1,3,6-trisulphonic acid), the pH sensing indicator. Increase of glucose concentration indicated that acidification (ΔpH -0.4) inside the nanoreactor was observed for the FEx-GOx-HRP and the FEx-1, but a transmembrane pH gradient was low. Activation of the assembled electron transport chain of the FEx-1 membrane was triggered by addition of the reduced DTT, which reduces the coenzyme Q1 and is utilized for bo3 oxidase activity, resulting in oxidation of the reduced Q1 (Q1H) by the bo3 oxidase. The protons were pumped into the vesicles after each fusion, resulting in ΔpH = -1.6 after 90 minutes. This established sufficient proton motive power (PMF) to induce the ATP synthesis. In other words, it was confirmed that the reconstituted protein can perform its full function through the activity of the bo3 oxidase proton pump of the fused exosome nanoreactor.

### 4-2. ATP activity

A standard calibration curve was drawn according to a ATP determination kit A22066 (Invitrogen) protocol. A standard solution containing 8.9 mL of H₂O and 0.5 mL of a reaction buffer (500 mM tricine buffer (pH 7.8), 100 mM of MgSO₄, 2 mM of EDTA, and 2 mM of sodium azide), 0.5 mL of 0.1 M DTT, 0.5 mL of 10 mM D-luciferin, and 2.5 µL of 5 mg/mL firefly luciferase were prepared. Since the synthesized ATP emits light when converted to pyrophosphate and adenosine monophosphate by luciferase, luminescence was measured using a spectrophotometer (TECAN, Morrisville, NC, USA). The results thereof were shown in FIG. 9(B). The ATP production with addition of glucose-DTT (1.31 ± 0.05 ATP µmol/mg) increased relatively quickly, compared to the ATP production with addition of glucose or DTT alone. Contrary to this, the ATP production was barely detectable in the absence of glucose and DTT.

FIG. 11 shows a degree of luminescence when the individual components (GOx, HRP, ATP synthase, and bo3 oxidase) prepared according to Comparative Example 2 was directly loaded into the exosome and a degree of luminescence when the fused exosome prepared according to Example 2 produced the ATP. It was confirmed that the ATP production in the fused exosome according to the present invention was significantly increased compared to the case of encapsulating the enzyme directly.

### [Experimental Example 5] Confirmation of spheroid penetration effect of fused exosome

### 5-1. Preparation of hypoxia-induced spheroid containing fused exosome

A MCF-10A (30 µL, 10⁵ cells/mL) cell suspension was dispensed onto a Petri plate and maintained in an inverted state. A bottom of the plate was filled with 10 mL of PBS to prevent evaporation and maintain humidity. The spheroid was cultured by aspirating ~5 µL of a medium every 24 hours and replacing it with 10 µL of a fresh medium. Twenty-four hours after replacing with 10 µL of the fresh cell medium containing a FEx-1 nanoreactor (206 µg), the spheroid was washed with the PBS. Afterwards, 30 µL of a cell medium containing 600 µM of ADP and 5 mM of MgCl₂ was added thereto, followed by addition of 60 µM of coenzyme Q1 and 3 mM of DTT (Dithiothreitol). After 24 hours of culturing, the spheroid was treated with 30 µM of TBHP (tert-butyl hydroperoxide) for 3 hours to prepare a hypoxia-induced spheroid.

### 5-2. Measurement of ATP activity

ATP production was measured by luminescence using a luciferin-luciferase assay. A standard calibration curve was obtained by varying an amount of the ATP in the range of 0 to 1000 nM and monitoring a luminescence intensity (from luciferin to oxyluciferin). The ATP activity (%) was determined based on the total amount of ATP present under a normal condition. The results thereof were shown in FIG. 12. It could be observed that the ATP activity was decreased about 13-folds under a hypoxic condition, but the ATP activity level when glucose-DTT was added to the spheroid having the FEx-1 nanoreactor loaded therein was recovered to 81.1 ± 5.9% close to the level of the positive control group without hypoxia.

### 5-3. Measurement of cell viability

After induction of hypoxia, cell viability of spheroid was measured using a LIVE/DEAD Viability/Cytotoxicity Kit for mammalian cells (cat# L3224, Thermo Fisher Scientific). 8 µM of calcein-AM and 16 µM of ethidium homodimer-1 were mixed in PBS, and 25% of the volume of a hanging drop containing the hypoxia-induced spheroid was replaced with this mixture. After incubation for 45 minutes, the spheroid was washed three times with fresh PBS to remove excess dye. The cell viability was measured using calcein-AM (λₑₓ = 490 nm and λₑₘ = 520 nm) and was shown in FIG. 13. The data represent the mean ± SD of n = 3 independent experiments.

It could be observed that under hypoxic condition, the cell viability was decreased by about 6-folds, but when glucose-DTT was added to the spheroid having the FEx-1 nanoreactor loaded therein, the cell viability was recovered to 93.7 ± 4.5% close to the level of the positive control group without hypoxia.

### 5-4. Measurement of oxidative stress (Reactive oxygen species, ROS) level

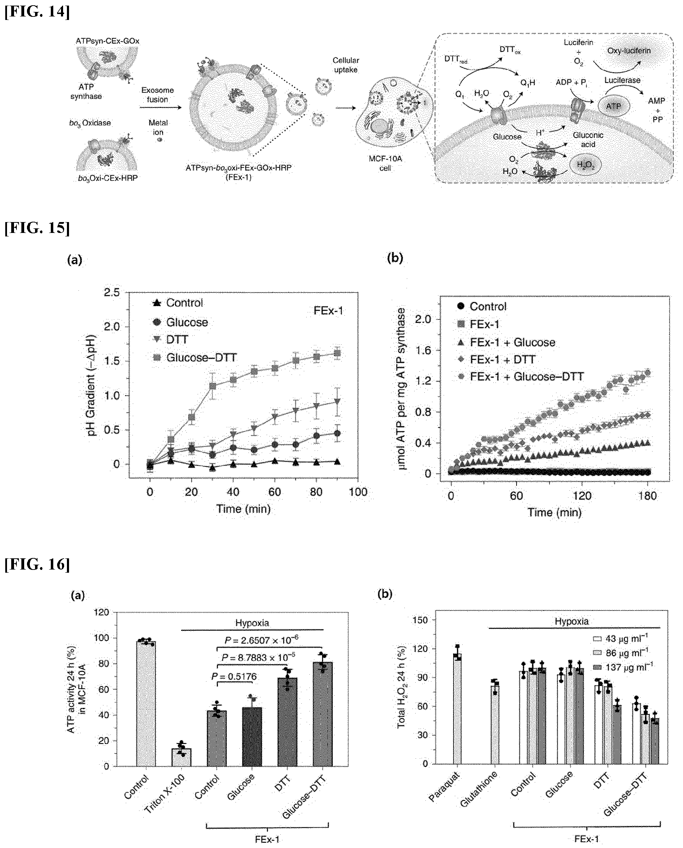

For positive and negative controls for ROS measurement, each of the spheroids was treated with 30 µM of paraquat and 50 µM of glutathione for 1 hour, washed twice with PBS, and then was incubated together with 100 µL of dichlorodihydrofluorescein diacetate at 37 °C for 1 hour. Afterwards, the spheroids were gently washed with the PBS to measure the fluorescence intensity using a spectrophotometer (TECAN, Morrisville, NC, USA) with excitation at 485 nm and emission at 535 nm. The data represent the mean ± SD of n = 3 independent experiments. It could be confirmed that as shown in FIG. 14, when treated with FEx-1 nanoreactor and glucose-DTT, the ROS in spheroids was reduced by about 2-folds, which shows that the nano-vesicle reactor according to the present invention can provide an effect of oxidative stress removal.

It was confirmed by the above that the vesicle nanoreactor according to the present invention can reduce cell damage caused by hypoxia observed under various disease conditions and regulate metabolic activity of the cell. In addition, it can be expected from the results of deep penetration into the spheroid core that the nano-vesicle reactor of the present invention can improve performance of the biocatalyst by penetrating deep into the tumor tissue without leaching of the loaded enzyme.

## Claims

1. A composition for producing a nano-vesicle reactor, **characterized by** comprising a first vesicle containing a first receptor on its surface and a second vesicle containing a second receptor on its surface, wherein the first receptor and the second receptor are bound with each other through a ligand system so that the first vesicle and the second vesicle are fused to form the nano-vesicle reactor.

2. The composition for producing the nano-vesicle reactor according to claim 1,
wherein the first receptor and the second receptor are independently with each other selected from the group consisting of CD9, CD63, CD81, CD82, CD147, CD107a, CD151, CD13, CD26, ITGA3, ITGA6, ITGB1, ITGB4, ADAM10, CD31B, EGFR, EpCAM, HSP70, HSP9, PSMA, and PSA.

3. The composition for producing the nano-vesicle reactor according to claim 1,
wherein the ligand system contains a coordination-bonding compound.

4. The composition for producing the nano-vesicle reactor according to claim 3,
wherein the coordination-bonding compound is an aromatic compound containing a polar substituent group.

5. The composition for producing the nano-vesicle reactor according to claim 4,
wherein the polar substituent group includes a hydroxyl group or a carboxylic acid group.

6. The composition for producing the nano-vesicle reactor according to claim 1,
wherein the ligand system further form a supramolecular complex with multivalent metal ion.

7. The composition for producing the nano-vesicle reactor according to claim 1,
wherein the first vesicle and the second vesicle are an extracellular vesicle.

8. The composition for producing the nano-vesicle reactor according to claim 1,
wherein the first vesicle and the second vesicle contains enzymes different with each other.

9. The composition for producing the nano-vesicle reactor according to claim 1,
wherein the first vesicle and the second vesicle have an average diameter of 10 to 1000 nm.

10. A nano-vesicle reactor-containing emulsion comprising the composition for producing the nano-vesicle reactor according to claim 1, in a aqueous phase inside the emulsion.

11. The nano-vesicle reactor-containing emulsion according to claim 10,
wherein the emulsion has an average diameter of 2 to 100*µ*m.

12. A cell comprising the nano-vesicle reactor according to claim 1.

13. The cell according to claim 12,
wherein the nano-vesicle reactor is used for generating energy.

14. The cell according to claim 12,
wherein the first vesicle contained in the nano-vesicle reactor includes ATP synthase, and the second vesicle includes bo3 oxidase.

15. The cell according to claim 12,
wherein the nano-vesicle reactor further comprises a drug.

16. A method for producing a nano-vesicle reactor, comprising the steps of:
(S1) preparing a first vesicle solution containing a first receptor on a surface of the vesicle;
(S2) preparing a second vesicle solution containing a second receptor on a surface of the vesicle;
(S3) preparing a mixed solution by mixing the first vesicle solution and the second vesicle solution; and
(S4) mixing the mixed solution with a multivalent metal ion to fuse the first vesicle and the second vesicle.

17. The method for producing the nano-vesicle reactor according to claim 16,
wherein the first vesicle solution and the second vesicle solution of the steps (S 1) and (S2) further comprise the step of treating them with a coordination-bonding compound independently with each other.
